(19) Europäisches Patentamt
European Patent Office
Office européen des brevets

(11) **EP 4 112 067 A1**

(12) **EUROPEAN PATENT APPLICATION**
published in accordance with Art. 153(4) EPC

(43) Date of publication:
**04.01.2023 Bulletin 2023/01**

(21) Application number: **20895446.1**

(22) Date of filing: **02.12.2020**

(51) International Patent Classification (IPC):
**A61K 38/00** *(2006.01)*    **A61P 9/00** *(2006.01)*

(52) Cooperative Patent Classification (CPC):
**A61P 9/10; A61K 38/00; A61K 38/44;**
**A61K 38/446; A61P 9/00; C12N 9/0089;**
C12Y 115/01001

(86) International application number:
**PCT/CN2020/133378**

(87) International publication number:
**WO 2021/110049 (10.06.2021 Gazette 2021/23)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **04.12.2019 CN 201911230383**

(71) Applicant: **Carry Health Biopharmaceuticals**
**(Hangzhou) Co., Ltd.**
**Hangzhou, Zhejiang 310026 (CN)**

(72) Inventor: **MENG, Fanguo**
**Hangzhou, Zhejiang 310026 (CN)**

(74) Representative: **Kador & Partner PartG mbB**
**Corneliusstraße 15**
**80469 München (DE)**

Remarks:
The complete document including Reference
Table(s) and the Sequence Listing(s) can be
downloaded from the EPO website

(54) **APPLICATION OF MANGANESE-TYPE HIGH-STABILITY SUPEROXIDE DISMUTASE IN PREVENTION OR TREATMENT OF STROKE**

(57)    The present invention relates to the use of a manganese superoxide dismutase with high stability and a pharmaceutical composition thereof in the prevention or treatment of cerebral stroke. The amino acid sequence of the manganese superoxide dismutase with high stability of the present invention is set forth in SEQ ID NO: 4. The manganese superoxide dismutase with high stability provided by the present invention can significantly prevent and reduce the cerebral injury after cerebral stroke, and has good prevention and treatment effects on cerebral stroke.

EP 4 112 067 A1

**Description**

TECHNICAL FIELD

The present invention belongs to the field of biomedicine, and specifically relates to the prevention or treatment of cerebral stroke.

BACKGROUND ART

Cerebral stroke, including ischemic cerebral stroke and hemorrhagic cerebral stroke, is a disease of brain tissue in which blood cannot flow into the brain normally due to blood vessel blockage or blood vessel rupture and bleeding. Ischemic cerebral stroke accounts for 70% of strokes and the stenosis and occlusion of the carotid arteries and vertebral arteries can cause ischemic cerebral stroke. After a cerebral stroke occurs, it will cause damage to the brain tissue, and severe cases are life-threatening. During ischemia and reperfusion, mitochondrial damage, calcium overload, free radical accumulation, inflammatory response, etc. may occur. Among them, the generation of excessive free radicals in the brain leads to an imbalance in the oxidation-antioxidant system, which is an important aspect. Free radicals cause peroxidation of lipids, proteins and nucleic acids, leading to damage and death of nerve cells. In addition, free radicals can also cause brain tissue damage through signal transduction, cell apoptosis, etc.

The drugs currently used to treat free radical damage include free radical scavengers (such as edaravone, etc.) and neuroprotectants (such as butylphthalide, etc.). These drugs are mainly small molecular substances and are used for free radical scavenging and post-injury protection. These drugs have certain effects on the treatment of stroke, but also have side effects. In addition, intravascular stent implantation and plaque removal surgery can prevent the occurrence of stroke in some patients. Therefore, there is still a need for drugs with good therapeutic effects for cerebral stroke.

SUMMARY OF THE INVENTION

The inventors of the present application have investigated the potential effects of superoxide dismutase on cerebral stroke, and proposed a new approach to prevent and treat the symptoms of cerebral stroke.

Specifically, the present invention provides the use of a manganese superoxide dismutase with high stability (MS-SOD) in the preparation of a medicament for preventing or treating cerebral stroke, wherein the amino acid sequence of the manganese superoxide dismutase with high stability is set forth in SEQ ID NO: 4.

Preferably, the medicament is administered by oral or injection route. More preferably, the injection is intravenous injection or intraperitoneal injection.

Preferably, the dosage form of the medicament is a tablet, a capsule, a powder injection, an injection or an aerosol.

Preferably, the medicament further comprises one or more pharmaceutically acceptable excipients, such as a diluent, a binder, a wetting agent, a disintegrating agent, a solvent and/or a buffer, and the like. These excipients are prepared into medicaments in a manner well known in the art with MS-SOD, and the dosages thereof are also known to those skilled in the art.

The present invention also provides a pharmaceutical composition capable of preventing or treating cerebral stroke, which consists of an active ingredient and pharmaceutically acceptable excipient(s), wherein the active ingredient is a manganese superoxide dismutase with high stability, and the amino acid sequence thereof is set forth in SEQ ID NO: 4.

Preferably, the activity of the superoxide dismutase in the pharmaceutical composition is 200U-200000U/day, preferably 2000U-200000U/day.

Preferably, the pharmaceutically acceptable excipient is a diluent, a binder, a wetting agent, a disintegrating agent, a solvent and/or a buffer, and the like.

In a third aspect, the present invention provides a method for preventing or treating cerebral stroke, comprising administering the manganese superoxide dismutase with high stability of the present invention or a pharmaceutical composition thereof to a person in need. Further, the method of administration is oral administration or injection administration at a dose of 200U-200000U/day, preferably 2000U-200000U/day.

In a fourth aspect, the present invention provides the use of a manganese superoxide dismutase with high stability or a pharmaceutical composition thereof in preventing or treating cerebral stroke.

Preferably, the cerebral stroke of the present invention is ischemic cerebral stroke or hemorrhagic cerebral stroke.

The manganese superoxide dismutase with high stability provided by the present invention can significantly reduce the cerebral injury after stroke, and has good improvement and therapeutic effects on the symptoms of cerebral stroke.

DESCRIPTION OF THE DRAWINGS

Figure 1 is a picture showing the cerebral sections of the mice in the model group, the MS-SOD1 group and the MS-SOD2 group. Among them, the model group is a middle cerebral artery occlusion model group; after intragastrical administration with SOD for 4 weeks (3 U/day/g body weight), the mice of the MS-SOD1 group were subjected to establish MCAO model; after establishing MCAO model, the mice of the MS-SOD2 group were intragastrically administered with SOD (600 U) once when they waked up from the anesthesia.

Figure 2 shows the area ratios of cerebral infarction in mice.

Figure 3 shows neurological severity scores in mice.

Figure 4 shows the Western blot assay results of intestinal tight junction proteins in mice.

Figure 5 is a phenotype chart showing the cerebral ischemia in zebrafish before and after MS-SOD treatment, and the dotted area is the cerebral ischemic location.

Figure 6 shows the improvement effect of MS-SOD on cerebral ischemia in zebrafish (compared with the model control group, ***$p < 0.001$).

Figure 7 is a phenotype chart showing the cerebral hemorrhage in zebrafish after MS-SOD treatment.

Figure 8 shows the improvement effect of MS-SOD on cerebral hemorrhage in zebrafish (compared with the model control group, *$p < 0.05$).

Figure 9 is a trajectory chart showing the movement improvement effect of MS-SOD on cerebral hemorrhage.

Figure 10 shows the movement improvement effect of MS-SOD on cerebral hemorrhage (movement velocity, compared with the model control group, *$p < 0.05$).

Figure 11 shows the improvement effect of MS-SOD on blood flow volume of cerebral hemorrhage (blood flow volume, compared with the model control group, **$p < 0.01$, ***$p < 0.001$).

DETAILED DESCRIPTION OF THE INVENTION

The present invention will be further described below in conjunction with examples, but these examples do not constitute any limitation to the present invention.

After years of research, the applicant has found that MS-SOD can regulate the treatment target of stroke-related risk factors, neuroinflammation responses and post stroke complications. Accordingly, in this study, the applicant has evaluated and detected the situation of cerebral injury, neurological deficit and the like, by establishing a middle artery embolism ischemic model in mice, a cerebral ischemia model in zebrafish and a cerebral hemorrhage model in zebrafishes, thereby analyzing the prevention and treatment effects of MS-SOD on cerebral stroke.

Unless otherwise specified, the reagents and instruments used in the following examples are all conventional reagents and instruments in the art, and are commercially available. The experimental methods and scoring methods used in the following examples are all conventional methods in the art, and the selection and setting of parameters are also conventional choices. Those skilled in the art can implement the methods without any doubt and obtain the corresponding results based on the content described.

**Example 1: Preparation of a manganese superoxide dismutase with high stability (MS-SOD)**

Using the genome of *Thermus thermophilus* HB27 (purchased from the ATCC cell bank of the United States, accession number: *ATCC BAA-163*) as a template, the following primers were used to carry out the amplification to obtain the target gene: forward primer: 5'-aagaattcatgccgtacccgttcaagct-3' (SEQ ID NO: 1) and reverse primer: 5'-ctgtcgactcaggctttgttgaagaac-3' (SEQ ID NO: 2); the amplified product was recovered using a recovery kit (purchased from Sangon Biotech (Shanghai) Co., Ltd.), double-digested with enzymes EcoRI and Sal I and ligated into the plasmid vector pET28a(+) (purchased from Sangon Biotech (Shanghai) Co., Ltd.) which was double-digested with the same enzymes. The resulting recombinant plasmid was transformed into competent *E. coli* BL21 (DE3) (purchased from Sangon Biotech (Shanghai) Co., Ltd.). The strain with the correct MS-SOD nucleotide sequence was screened by sequencing, and the strain was cultured to obtain MS-SOD protein. The nucleotide sequence of the gene encoding MS-SOD is as follows:

atgccgtacccgttcaagcttcctgacctaggctacccctacgaggccctcgagccccacattgacgccaagaccatgg

agatccaccaccagaagcaccacggggcctacgtgacgaacctcaacgccgccctggagaagtaccccctacctcca

cggggtggaggtggaggtcctcctgaggcacctcgccgcccttccccaggacatccagaccgccgtgcgcaacaac

gggggcgggcacctgaaccacagcctcttctggaggctcctcaccccccggggggggccaaggagcccgtggggggag

ctgaagaaggccattgacgagcagttcggggggcttccaggccctcaaggagaagctcacccaggcggccatgggcc

ggttcggctcgggctgggcctggctcgtgaaggacccccttcggcaagctccacgtcctctccaccccaaccaagaca

accccgtgatggagggcttcaccccatcgtgggcattgacgtctgggagcacgcctactacctcaagtaccagaacc

gccgggccgattacctccaggccatctggaacgtcctcaactgggacgtggccgaggagttcttcaataaagcctga (SEQ ID NO: 3).

The amino acid sequence of MS-SOD is as follows:

MPYPFKLPDLGYPYEALEPHIDAKTMEIHHQKHHGAYVTNLNAALEKYPYLHGVEVE

VLLRHLAALPQDIQTAVRNNGGGHLNHSLFWRLLTPGGAKEPVGELKKAIDEQFGGF

QALKEKLTQAAMGRFGSGWAWLVKDPFGKLHVLSTPNQDNPVMEGFTPIVGIDVWE

HAYYLKYQNRRADYLQAIWNVLNWDVAEEFFKKA (SEQ ID NO: 4).

**Example 2: Experiments of MS-SOD on cerebral ischemia model in mice**

**1. Source of animals**

Male C57BL/6J wild-type mice were all purchased from Guangdong Experimental Animal Center. The male C57BL/6J wild-type mice were 8 weeks old, when they were subjected to establish middle cerebral artery occlusion(MCAO) model.

**2. Grouping and treatment of mice**

According to different intervention means of MS-SOD, the mice were divided into the following groups:

(1) Control group (10 mice): healthy mice aged 8 weeks;

(2) Model group (10 mice): middle cerebral artery occlusion model group without intervention of MS-SOD; the process of model establishment is shown in section 3 below;

(3) MS-SOD1 group (10 mice): mice aged 4 weeks were fed with drinking water supplemented with MS-SOD for 4 weeks (3 U/day/g body weight), and then subjected to establish middle cerebral artery occlusion model;

(4) MS-SOD2 group (10 mice): after establishing middle cerebral artery occlusion model, the mice were intragastrically administered with MS-SOD 600 U once when they waked up from the anesthesia.

**3. Establishment of middle cerebral artery occlusion model**

The process of MCAO model establishment in the mice of groups (2) - (4) was as follows: the mice were weighed and anaesthetized with tribromoethanol (0.2 ml / 10 g body weight); then a midline incision of about 1 cm in the neck was made and the tissue was carefully dissected to expose the right carotid trigonum; the right common carotid artery, the external carotid artery (dissected as far up as possible to the anterior cranial bifurcation) and the internal carotid artery were separated carefully; the proximal side of common carotid artery was ligated (slipknot), the small branches of the external carotid artery were broken by coagulation with the tip of electric coagulator, the proximal side (slipknot) and the distal side (fast knot) of external carotid artery were both ligated; the external carotid artery was cut and a thread (with an appropriate size selected according to the body weight of the mouse) was inserted in the direction of the external carotid artery, and the insertion was stopped when slight resistance was felt (approximately when the thread was marked (1 cm from the head) at bifurcation of the common carotid artery); the thread was fixed, and the skin was then sutured; the common carotid artery was opened after the middle cerebral artery was occluded for 60 minutes, and the cerebral infarction detection was performed at 72 hours after ischemia reperfusion. During the operation, a thermostatic blanket was used to maintain the core body temperature of the mouse at 37 + 0.5 °C, until the mouse was awakened after the anesthesia effect, thereby preventing the hypothermic brain protection from affecting the establishment of the cerebral ischemia model in mice.

**4. Experimental methods**

**(1) Modified Neurological Severity Score**

The procedure was performed on each group of mice. The mental state, fur luster and brightness, autonomous activity, body movement, ingestive situation and changes in body weight of all mice were observed at 24 hours after MCAO model establishment. Modified Bederson and Longa method was used to evaluate behavioral function on a 4 - point scale. The mice in groups 2 - 4 were scored by double blind method. Score 0: no obvious nervous functional deficiency symptoms; score 1: inability to fully extend the paralyzed side forelimb and when the mouse tail was lifted 30 cm off the ground, the left forelimb showed wrist flexion, elbow flexion, shoulder internal rotation or a combination thereof; score 2: the rat turned in a circle to the paralyzed side when walking; score 3: when walking, the body of rat tilted to the paralyzed side or tilted to the left side when the shoulder was gently pushed; score 4: inability to walk or loss of consciousness.

**(2) Evaluation of cerebral infarction area in mice**

At 24 hours after MCAO model establishment, the head was cut off and the brain was taken after anesthesia. The brain was frozen at -20°C for 15 min and then coronal sections with a thickness of 1.5 mm were made. The brain sections were quickly placed in 1% 2,3,5-triphenyltetrazolium chloride (TTC) in phosphate buffer, and incubated at 37°C in the dark for about 10 minutes. The normal brain tissue was stained dark red and the cerebral infarction territory was not stained (grayish-white). The infarction area of each territory was measured by ImageJ software. For the model, the applicant formulated a formula of edema corrected infarction area: Infarction area = direct injury area - (area of the ipsilateral hemisphere of the body - area of the contralateral hemisphere of the body). The total infarction area was calculated by integrating the infarction territories between sections.

**(3) Detection of tight junction proteins in the intestinal barrier**

Intestinal tissue of each mouse in each group was obtained and frozen. The frozen intestinal tissue was homogenized in a mammalian tissue lysis buffer on ice, and centrifuged at 1,4000 rpm for 15 minutes to isolate proteins. Protein concentration was determined using the BCA method. An equal amount of each intestinal tissue homogenate sample was loaded onto 12 % SDS-PAGE and transferred to a polyvinylidene fluoride membrane (Millipore Corporation, Bedford, MA, USA). The membrane was blocked with 5% skimmed milk in TBST buffer at room temperature for 60 minutes, and then incubated with the primary antibodies against occludin, claudin-4, and ZO-1 (Zona occludens 1) overnight at 4 °C. The membrane was then incubated with the appropriate secondary antibody conjugated with horseradish peroxidase (Santa Cruz Biotechnology Inc, Santa Cruz, Canada) for 1 hour at 37 °C, the signal was visualized using an enhanced chemiluminescence (ECL) detection system (Amersham), and the intensity of spectral band was determined by ImageJ software.

**(4) Statistical analysis**

Finally, the differences of different intervention conditions and MCAO model establishment on the degree of cerebral injury and neurological severity in mice were analyzed. All data were expressed as mean $\pm$ standard deviation. SPSS 20.0 software was used for statistics, and repeated measurement analysis of variance was used for statistical analysis. Bonferroni method was used for comparison between groups, $P<0.05$ was considered to indicate a statistically significant difference.

**5. Results**

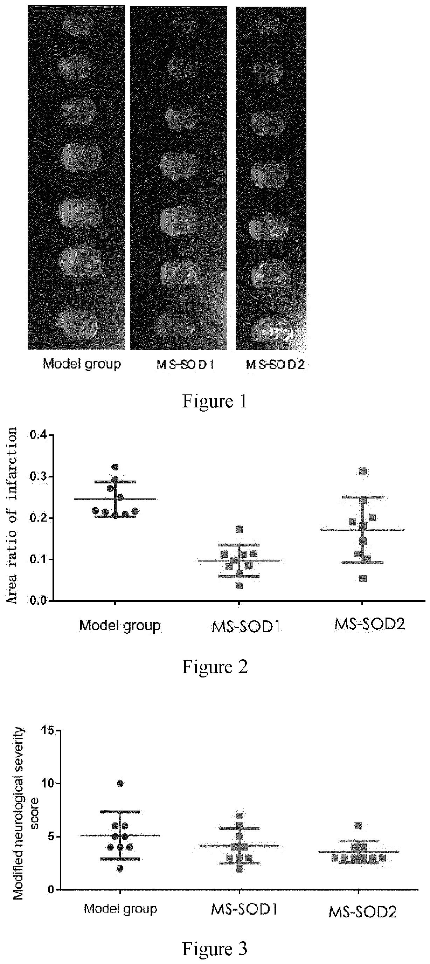

The cerebral sections of mice in each group were shown in Figure 1. Compared with the mice in the model group, the area of cerebral injury was significantly reduced (Mann-Whitney U Analysis, $P=0$) after preoperative long-term intragastrical intervention with MS-SOD (MS-SOD1 group), while the neurological severity score of the mice was not significantly improved (Mann-Whitney U Analysis, $P = 0.281$) (Figure 2 and Figure 3). Compared with the mice in the model group, the area of cerebral injury was significantly reduced (Mann-Whitney U Analysis, $P=0.015$) after postoperative short-term intragastrical intervention with MS-SOD (MS-SOD2 group), and the neurological severity score (mNSS) was significantly reduced too (Mann-Whitney U Analysis, $P=0.05$) (Figure 2 and Figure 3). The results showed that long-term intragastrical administration before stroke and intragastrical administration after stroke can prevent and treat the damage of cerebral tissue, and also have a certain recovery effect on neurological function injury.

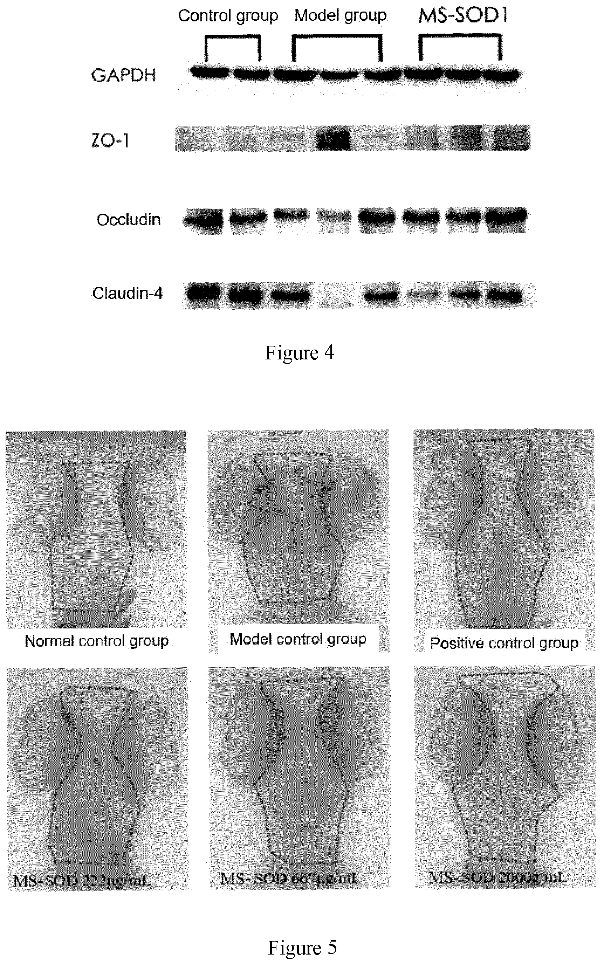

Intestinal tight junction proteins were detected by Western Blot in cecum samples of three mice that respectively randomly selected from the model group, the control group and the MS-SOD1 group. The results were shown in Figure 4 (Figure 4 shows the detection results of ZO-1, occludin and claudin-4 in the intestinal tight junction proteins). Compared with the mice in the control group, the expression levels of intestinal tight junction proteins in the mice of the model group and the MS-SOD1 group were decreased. After analyzing the gray value of bands with Image J software, the expression of cecal occludin in the mice of the model group was lower than that of the control group (T test, $P=0.013$), and the expression of claudin-4 was lower than that of the control group (T test, $P=0.07$). The expression of cecal occludin in the mice of the MS-SOD1 group was lower than that of the control group (T test, $P=0.018$).

**Example 3: Experiments of MS-SOD on treating ischemic cerebral stroke model in zebrafish**

**1. Source of animals**

Zebrafishes were provided by Hunter Biotechnology, Inc.

**2. Grouping of animals**

| | |
|---|---|
| Experiment group 1 | normal control group |
| Experiment group 2 | model control group |
| Experiment group 3 | positive control drug aspirin 50 $\mu$g/mL |
| Experiment group 4 | MS-SOD 222 $\mu$g/mL |
| Experiment group 5 | MS-SOD 667 $\mu$g/mL |
| Experiment group 6 | MS-SOD 2000 $\mu$g/mL |

Wherein the specific activity of MS-SOD was 5 U/$\mu$g

**3. Model establishment**

Melanin allele mutated Albino strain zebrafish at 2 days post fertilization (2 dpf) was administered with an aqueous ponatinib solution (1 $\mu$g/mL) for 18 hours to establish a cerebral ischemia model in zebrafish.

**4. Experimental method**

180 melanin allele mutated Albino strain zebrafishes at 2 days post fertilization (2 dpf) were randomly selected. 30 zebrafishes were treated in each well (experiment group), and aqueous MS-SOD solutions with concentrations of 222, 667 and 2000 $\mu$g/mL were administrated respectively, the concentration of positive control drug aspirin was 50

$\mu$g/mL, and the normal control group and the model control group were set at the same time. The volume of each well was 3 mL. After MS-SOD treatment for a period of time, except for the normal control group, all the other experiment groups were simultaneously administrated with aqueous ponatinib solution to induce the cerebral ischemia model in zebrafish. After 18 hours of co-treatment with MS-SOD and ponatinib, 20 fish were randomly selected from each well and stained with o-Dianisidine. After staining, photos were taken and data were collected to count the number of zebrafish with cerebral ischemia and calculate the incidence of cerebral ischemia in zebrafish of each experiment group.

**5. Experimental results**

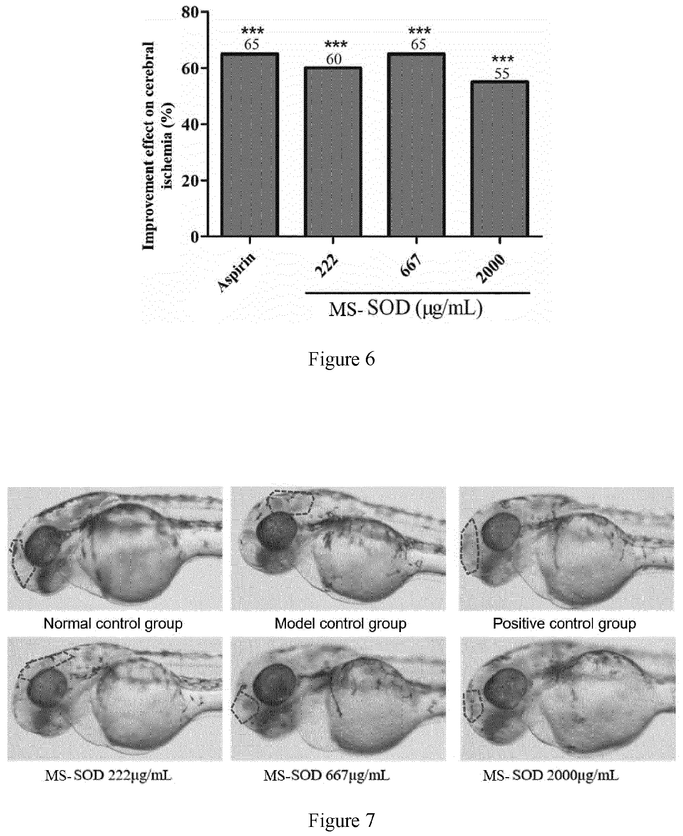

Compared with 0% (0/20) in the normal control group, the incidence of cerebral ischemia in the zebrafishes of the model control group was 100% (20/20), $p < 0.001$, indicating that the cerebral ischemia model in zebrafish was successfully established. Compared with the model control group, the incidence of cerebral ischemia in the zebrafishes of the positive control drug aspirin 50 $\mu$g/mL group was 35% (7/20), $p < 0.001$, the improvement effect on cerebral ischemia was 65%, indicating that aspirin had a significant improvement effect on cerebral ischemia in zebrafish. Compared with 100% (20/20) in the model control group, when the concentrations of MS-SOD were 222, 667 and 2000 $\mu$g/mL, the incidences of cerebral ischemia were 40% (8/20), 35% (7/20) and 45% (9/20) respectively, $p < 0.001$ & $p < 0.001$ & $p < 0.001$, the improvement effects on cerebral ischemia were 60%, 65% and 55% respectively, indicating that MS-SOD had a significant therapeutic effect on cerebral ischemia in zebrafish under the experimental concentration conditions. The specific results are shown in Table 1, Figure 5 and Figure 6.

Table 1: Improvement effect of MS-SOD on cerebral ischemia in zebrafish (n=20)

| Group | Concentration ($\mu$g/mL) | Number of zebrafish with cerebral ischemia | Incidence of cerebral ischemia (%) | Improvement effect on cerebral ischemia (%) |
|---|---|---|---|---|
| Normal control group | - | 0 | 0 | - |
| Model control group | - | 20 | 100 | - |
| Aspirin | 50 | 7 | 35*** | 65*** |
| MS-SOD | 222 | 8 | 40** | 60*** |
| | 667 | 7 | 35*** | 65*** |
| | 2000 | 9 | 45*** | 55*** |

Compared with the model control group: ***$p < 0.001$.

**Example 4: Evaluation of improvement effect of MS-SOD on cerebral hemorrhage**

**Source of animals:**

Zebrafishes were provided by Hunter Biotechnology, Inc.

**1. Concentration groups**

| | |
|---|---|
| Experiment group 1 | normal control group |
| Experiment group 2 | model control group |
| Experiment group 3 | positive control drug icariin 30 $\mu$M |
| Experiment group 4 | MS-SOD 222 $\mu$g/mL |
| Experiment group 5 | MS-SOD 667 $\mu$g/mL |
| Experiment group 6 | MS-SOD 2000 $\mu$g/mL |

Wherein the specific activity of MS-SOD was 5 U/$\mu$g

**2. Model establishment**

1 dpf wild-type AB strain zebrafish was administered with an aqueous simvastatin solution (0.5 $\mu$M) for 18 hours to establish a cerebral hemorrhage model in zebrafish.

**3. Experimental method**

180 wild-type AB strain zebrafishes at 1 day post fertilization (1 dpf) were randomly selected and put in a six-well plate, 30 per well (experiment group). Simvastatin was used to induce a cerebral hemorrhage model in zebrafish. Aqueous MS-SOD solutions with concentrations of 222, 667 and 2000 $\mu$g/mL were administrated respectively, the concentration of positive control drug icariin was 30 $\mu$M, and the normal control group and the model control group were set at the same time. The volume of each well was 3 mL. After 18 hours of co-treatment with MS-SOD and simvastatin, 30 zebrafishes from each group were selected and observed under a microscope, photos were taken and saved. The number of zebrafish with cerebral hemorrhage was counted, and the incidence of cerebral hemorrhage in zebrafish of each experiment group was calculated.

**4. Experimental results**

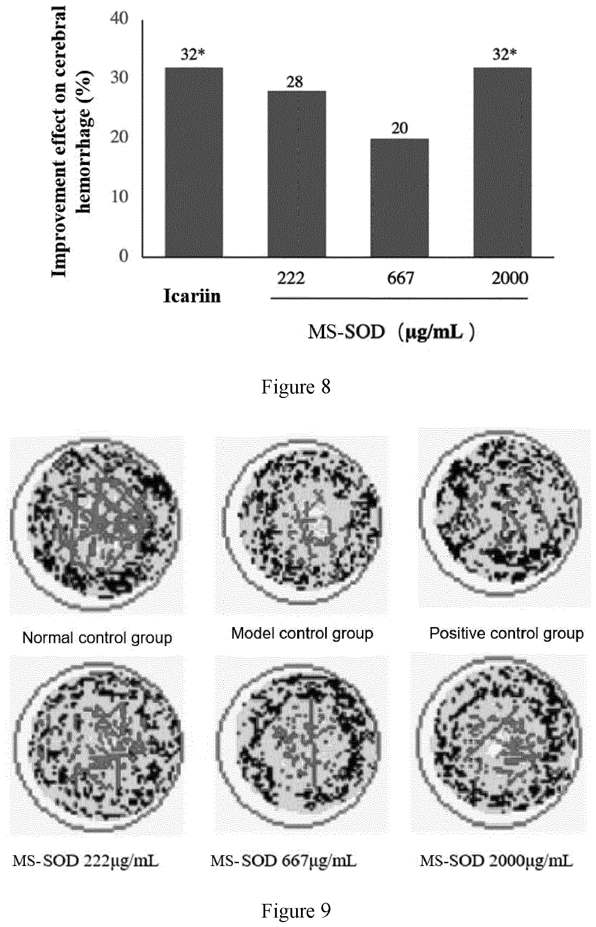

Results: the incidence of cerebral hemorrhage in the zebrafishes of the model control group was 83% (25/30), the incidence of cerebral hemorrhage in the zebrafishes of the positive control drug icariin 30 $\mu$M group was 57% (17/30), the improvement effect on cerebral hemorrhage was 32%; when the concentrations of MS-SOD were 222, 667 and 2000 $\mu$g/mL, the incidences of cerebral hemorrhage were 60% (18/30), 67% (20/30) and 57% (17/30) respectively, the improvement effect on cerebral hemorrhage was 28%, 20% and 32% respectively, indicating that MS-SOD had a significant improvement effect on cerebral hemorrhage in zebrafish. The specific results are shown in Table 2, Figure 7 and Figure 8.

Table 2: Improvement effect of MS-SOD on cerebral hemorrhage in zebrafish (n=30)

| Group | Concentration ($\mu$g/mL) | Number of zebrafish with cerebral hemorrhage | Incidence of cerebral hemorrhage (%) | Improvement effect on cerebral hemorrhage (%) |
|---|---|---|---|---|
| Normal control group | - | 0 | 0 | - |
| Model control group | - | 25 | 83 | - |
| Icariin | 30 $\mu$M | 17 | 56* | 32* |
| MS-SOD | 222 | 18 | 60 | 28 |
| | 667 | 20 | 67 | 20 |
| | 2000 | 17 | 57* | 32* |

Compared with the model control group: *$p < 0.05$.

**Example 5: Evaluation of behavioral improvement effect of MS-SOD on cerebral hemorrhage**

**Source of animals:**

Zebrafishes were provided by Hunter Biotechnology, Inc.

**1. Concentration groups**

Experiment group 1    normal control group
Experiment group 2    model control group
Experiment group 3    positive control drug icariin 30 $\mu$M
Experiment group 4    MS-SOD 222 $\mu$g/mL
Experiment group 5    MS-SOD 667 $\mu$g/mL
Experiment group 6    MS-SOD 2000 $\mu$g/mL

Wherein the specific activity of MS-SOD was 5 U/$\mu$g

## 2. Model establishment

5 dpf wild-type AB strain zebrafish was administered with an aqueous simvastatin solution (0.5 $\mu$M) for 18 hours to establish a cerebral hemorrhage model in zebrafish.

## 3. Experimental method

180 wild-type AB strain zebrafishes at 5 days post fertilization (5 dpf) were randomly selected and put in a six-well plate, 30 per well (experiment group). Aqueous MS-SOD solutions with concentrations of 222, 667 and 2000 $\mu$g/mL were administrated respectively, the concentration of positive control drug icariin was 30 $\mu$M, and the normal control group and the model control group were set at the same time. The volume of each well was 3 mL. After treatment of zebrafishes with samples for 4 hours, except for the normal control group, the other experiment groups were treated with simvastatin for 18 hours to establish the cerebral hemorrhage model. After the treatment, 10 zebrafishes from each group were randomly selected to measure the movement velocity (V) of zebrafish using a behavioral analyzer. The statistical significance of the movement velocity was used to evaluate the behavioral improvement effect of MS-SOD on cerebral hemorrhage. The formula of the behavioral improvement effect of MS-SOD on cerebral hemorrhage was as follows:

$$\text{Movement improvement effect (\%)} = \frac{V(\text{sample group}) - V(\text{model control group})}{V(\text{normal control group}) - V(\text{model control group})} \times 100\%$$

Statistical analysis was performed using variance analysis and Dunnett's T-test, and p<0.05 indicated a significant difference.

## 4. Experimental results

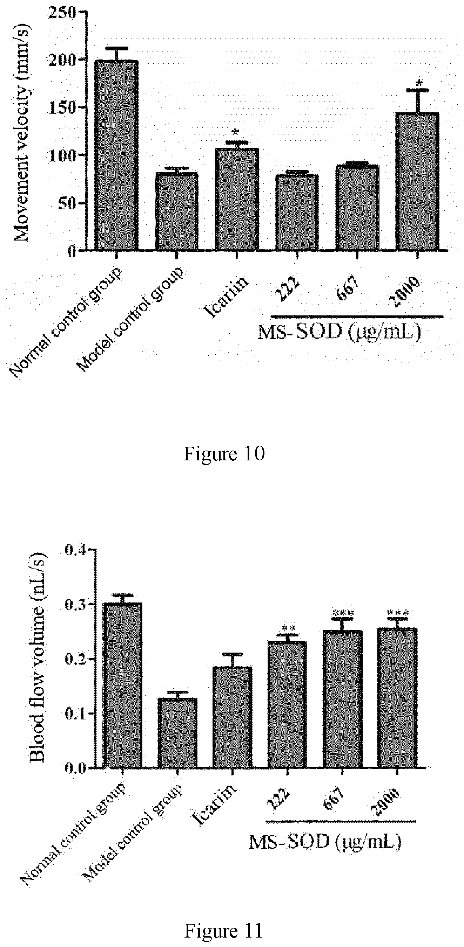

Results: compared with the normal control group (198 mm/s), the movement velocity of the zebrafishes in the model control group was 80 mm/s, p < 0.001, indicating that the model was successfully established; compared with the model control group (80 mm/s), the movement velocity of the zebrafishes in the positive control drug icariin 30 $\mu$M group was 106 mm/s, p < 0.05, the movement improvement effect was 22 %, indicating that the icariin had a significant improvement effect on the movement velocity of zebrafish after cerebral hemorrhage stroke; compared with the model control group, when the concentrations of MS-SOD were 222, 667 and 2000 $\mu$g/mL, the movement velocities of the zebrafishes were 78, 88 and 143 mm/s respectively, p > 0.05 & p > 0.05 & p < 0.05, the movement improvement effects were -2%, 7% and 53%, indicating that MS-SOD at 2000 $\mu$g/mL had a significant behavioral improvement effect on cerebral hemorrhage . The results are shown in Table 3, Figure 9 and Figure 10.

Table 3: Quantitative evaluation of the movement improvement effect of MS-SOD after cerebral ischemia (n=10)

| Group | Concentration ($\mu$g/mL) | Movement velocity (mm/s) (mean $\pm$ SE) | Movement improvement effect (%) |
|---|---|---|---|
| Normal control group | - | 198 $\pm$ 13 | - |
| Model control group | - | 80 $\pm$ 7 | - |
| Icariin | 25 | 106 $\pm$ 7* | 22* |

(continued)

| Group | Concentration ($\mu$g/mL) | Movement velocity (mm/s) (mean $\pm$ SE) | Movement improvement effect (%) |
|---|---|---|---|
| MS-SOD | 222 | 78 $\pm$ 4 | -2 |
| | 667 | 88 $\pm$ 3 | 7 |
| | 2000 | 143 $\pm$ 24* | 53* |

Compared with the model control group, * p <0.05.

**Example 6: Evaluation of improvement effect of MS-SOD on blood flow volume of cerebral hemorrhage**

**Source of animals:**

Zebrafishes were provided by Hunter Biotechnology, Inc.

**1. Concentration groups**

|   |   |
|---|---|
| Experiment group 1 | normal control group |
| Experiment group 2 | model control group |
| Experiment group 3 | positive control drug icariin 30 $\mu$M |
| Experiment group 4 | MS-SOD 222 $\mu$g/mL |
| Experiment group 5 | MS-SOD 667 $\mu$g/mL |
| Experiment group 6 | MS-SOD 2000 $\mu$g/mL |

Wherein the specific activity of MS-SOD was 5 U/$\mu$g

**2. Model establishment**

3 dpf wild-type AB strain zebrafish was administered with an aqueous simvastatin solution (0.5 $\mu$M) for 18 hours to establish a cerebral hemorrhage model in zebrafish.

**3. Experimental method**

180 wild-type AB strain zebrafishes at 3 days post fertilization (3 dpf) were randomly selected and put in a six-well plate, 30 per well (experiment group). Aqueous MS-SOD solutions with concentrations of 222, 667 and 2000 $\mu$g/mL were administrated respectively, the concentration of positive control drug icariin was 30 $\mu$M, and the normal control group (the zebrafishes were treated with water for fish) and the model control group were set at the same time. The volume of each well was 3 mL. After treatment of zebrafishes with samples for 4 hours, except for the normal control group, the other experiment groups were treated with simvastatin for 18 hours to establish the cerebral hemorrhage model. After the treatment, 10 zebrafishes from each group were randomly selected and placed in a heart rate and blood flow analysis system to record blood flow video of zebrafish, and analyze the blood flow volume (O) of zebrafish. The statistical significance of the blood flow volume was used to evaluate the improvement effect of MS-SOD on blood flow volume of cerebral hemorrhage. The formula of the improvement effect of MS-SOD on blood flow volume of cerebral hemorrhage was as follows:

$$\text{Blood flow volume improvement effect (\%)} = \left( \frac{O(\text{sample group}) - O(\text{model control group})}{O(\text{normal control group}) - O(\text{model control group})} \right) \times 100\%$$

Statistical analysis was performed using variance analysis and Dunnett's T-test, and p<0.05 indicated a significant difference.

**4. Experimental results**

Results:, compared with the normal control group (0.30 nL/s), the blood flow volume of the zebrafishes in the model control group was 0.13 nL/s, $p < 0.001$, indicating that the model was successfully established; the blood flow volume of the zebrafishes in the positive control drug icariin 30 $\mu$M group was 0.18 nL/s, $p > 0.05$, the movement improvement effect was 29%, indicating that the improvement effect of icariin on the blood flow volume of cerebral hemorrhage was not obvious; compared with the model control group, when the concentrations of MS-SOD were 222, 667 and 2000 $\mu$g/mL, the blood flow volumes of the zebrafishes were 0.23, 0.25 and 0.25 nL/s respectively, $p < 0.01$ & $p < 0.001$ & $p < 0.001$, the improvement effects on blood flow volume were 59%, 71% and 71% respectively, indicating that MS-SOD had a significant improvement effect on the blood flow volume of cerebral hemorrhage. The specific results are shown in Table 4 and Figure 11.

Table 4: Quantitative evaluation of the improvement effect of MS-SOD on the blood flow volume of cerebral hemorrhage (n=10)

| Group | Concentration ($\mu$g/mL) | Blood flow volume (nL/s) (mean $\pm$ SE) | Blood flow volume improvement effect (%) |
|---|---|---|---|
| Normal control group | - | 0.30 $\pm$ 0.016 | - |
| Model control group | - | 0.13 $\pm$ 0.014 | - |
| Icariin | 30$\mu$M | 0.18 $\pm$ 0.025 | 29 |
| MS-SOD | 222 | 0.23 $\pm$ 0.014** | 59** |
| | 667 | 0.25 $\pm$ 0.024*** | 71*** |
| | 2000 | 0.25 $\pm$ 0.019*** | 71*** |

Compared with the model control group, **$p < 0.01$, ***$p < 0.001$.

All documents mentioned in the present invention are cited as references in this application, and each document is individually cited as a reference. In addition, it should be understood that after reading the above content of the present invention, those skilled in the art can make various changes or modifications to the present invention, and these equivalent and alternative forms also fall within the scope defined by the claims of the present application.

**Claims**

1. Use of a manganese superoxide dismutase with high stability in the preparation of a medicament for preventing or treating cerebral stroke, wherein the amino acid sequence of the manganese superoxide dismutase with high stability is set forth in SEQ ID NO: 4.

2. The use according to claim 1, wherein the medicament is administered by oral or injection route; preferably, the injection is intravenous injection or intraperitoneal injection.

3. The use according to claim 1 or 2, wherein the dosage form of the medicament is a tablet, a capsule, a powder injection, an injection or an aerosol.

4. The use according to any one of claims 1 to 3, wherein the medicament further comprises one or more pharmaceutically acceptable excipients; preferably, the one or more pharmaceutically acceptable excipients is a diluent, a binder, a wetting agent, a disintegrating agent, a solvent and/or a buffer.

5. The use according to any one of claims 1 to 4, wherein the cerebral stroke is ischemic cerebral stroke or hemorrhagic cerebral stroke.

6. A pharmaceutical composition for preventing or treating of cerebral stroke, the pharmaceutical composition consists of an active ingredient and pharmaceutically acceptable excipient(s), wherein the active ingredient is a manganese superoxide dismutase with high stability, and the amino acid sequence thereof is set forth in SEQ ID NO: 4; preferably, the pharmaceutically acceptable excipient(s) is a diluent, a binder, a wetting agent, a disintegrating agent, a solvent and/or a buffer.

7. The pharmaceutical composition according to claim 6, wherein the activity of the superoxide dismutase in the pharmaceutical composition is 200U-200000U/day, preferably 2000U-200000U/day.

8. A method for preventing or treating of cerebral stroke, comprising administering a manganese superoxide dismutase with high stability or a pharmaceutical composition thereof to a person in need, wherein the amino acid sequence of the manganese superoxide dismutase with high stability is set forth in SEQ ID NO: 4; preferably, the method of administration is oral administration or injection administration; more preferably, the cerebral stroke is ischemic cerebral stroke or hemorrhagic cerebral stroke.

9. The method according to claim 8, wherein the dose of the manganese superoxide dismutase with high stability is 200U-200000U/day, preferably 2000U-200000U/day.

10. Use of a manganese superoxide dismutase with high stability or a pharmaceutical composition thereof for preventing or treating of cerebral stroke, wherein the amino acid sequence of the manganese superoxide dismutase with high stability is set forth in SEQ ID NO: 4.

Figure 1

Figure 2

Figure 3

Figure 4

Figure 5

Figure 6

| Normal control group | Model control group | Positive control group |
| MS-SOD 222μg/mL | MS-SOD 667μg/mL | MS-SOD 2000μg/mL |

Figure 7

Figure 8

Figure 9

Figure 10

Figure 11

## INTERNATIONAL SEARCH REPORT

| International application No. |
|---|
| **PCT/CN2020/133378** |

**A. CLASSIFICATION OF SUBJECT MATTER**

A61K 38/00(2006.01)i；ㅤA61P 9/00(2006.01)i

According to International Patent Classification (IPC) or to both national classification and IPC

**B. FIELDS SEARCHED**

Minimum documentation searched (classification system followed by classification symbols)

ㅤA61K; A61P

Documentation searched other than minimum documentation to the extent that such documents are included in the fields searched

Electronic data base consulted during the international search (name of data base and, where practicable, search terms used)

ㅤDatabase: DWPI, SIPOABS, CNABS, ISI Web of knowledge, EMBASE, NCBI PUBMED, CNKI, MEDLINE, Google Scholar, GenBank, EMBL, 中国专利生物序列检索系统, Chinese Patent Biological Sequence Retrieval System, Keywords: 超氧化物歧化酶, 锰, 脑卒中, 中风, 出血, 血栓, SOD, Mn-SOD, CNS, Manganese, stroke, brain, bleed, thrombosis, 对SEQ ID NO. 3, 4的检索, search on SEQ ID NO. 3 and 4

**C. DOCUMENTS CONSIDERED TO BE RELEVANT**

| Category* | Citation of document, with indication, where appropriate, of the relevant passages | Relevant to claim No. |
|---|---|---|
| X | WO 0028041 A1 (MICROBIOLOGICAL RESEARCH AUTHORITY et al.) 18 May 2000 (2000-05-18) <br> ㅤclaims, and description, page 8, paragraph 2, page 12, paragraphs 1-3 and page 17 | 6, 7 |
| Y | WO 0028041 A1 (MICROBIOLOGICAL RESEARCH AUTHORITY et al.) 18 May 2000 (2000-05-18) <br> ㅤclaims, and description, page 8, paragraph 2, page 12, paragraphs 1-3 and page 17 | 1-5 |
| Y | CN 109276711 A (HANGZHOU REDOX PHARMACEUTICAL TECHNOLOGY CO., LTD.) 29 January 2019 (2019-01-29) <br> ㅤclaims | 1-5 |

☐ Further documents are listed in the continuation of Box C.ㅤㅤㅤ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **15 December 2020** | **04 March 2021** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088** <br> **China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2020/133378**

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

   a.  ☑ forming part of the international application as filed:

         ☑ in the form of an Annex C/ST.25 text file.

         ☐ on paper or in the form of an image file.

   b.  ☐ furnished together with the international application under PCT Rule 13*ter*.1(a) for the purposes of international search only in the form of an Annex C/ST.25 text file.

   c.  ☐ furnished subsequent to the international filing date for the purposes of international search only:

         ☐ in the form of an Annex C/ST.25 text file (Rule 13*ter*.1(a)).

         ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

International application No.

**PCT/CN2020/133378**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **8-10**
   because they relate to subject matter not required to be searched by this Authority, namely:

   [1]  The subject matter of claims 8-10 relates to a method of treatment of the human or animal body by therapy and therefore does not warrant an international search according to the criteria set out in PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2020/133378**

| Patent document cited in search report | | | Publication date (day/month/year) | Patent family member(s) | | | Publication date (day/month/year) |
|---|---|---|---|---|---|---|---|
| WO | 0028041 | A1 | 18 May 2000 | EP | 1127136 | A1 | 29 August 2001 |
| | | | | JP | 2002529093 | A | 10 September 2002 |
| | | | | US | 2005255093 | A1 | 17 November 2005 |
| | | | | AU | 754883 | B2 | 28 November 2002 |
| | | | | CA | 2349496 | A1 | 18 May 2000 |
| | | | | AU | 1059400 | A | 29 May 2000 |
| | | | | GB | 9824282 | D0 | 30 December 1998 |
| | | | | US | 7470661 | B2 | 30 December 2008 |
| CN | 109276711 | A | 29 January 2019 | None | | | |

Form PCT/ISA/210 (patent family annex) (January 2015)